(19) Europäisches Patentamt / European Patent Office / Office européen des brevets

(11) **EP 2 069 141 B1**

## (12) EUROPEAN PATENT SPECIFICATION

(45) Date of publication and mention
of the grant of the patent:
**08.01.2014 Bulletin 2014/02**

(51) Int Cl.:
**B32B 25/10** (2006.01)

(21) Application number: **07814818.6**

(22) Date of filing: **12.09.2007**

(86) International application number:
**PCT/US2007/078245**

(87) International publication number:
**WO 2008/033903 (20.03.2008 Gazette 2008/12)**

(54) **AN ACTIVATABLE ZERO STRAIN COMPOSITE LAMINATE**

AKTIVIERBARES SPANNUNGSLOSES VERBUNDLAMINAT

MATÉRIAU STRATIFIÉ À TENSION ZÉRO SUSCEPTIBLE D'ACTIVATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **15.09.2006 EP 06120777**

(43) Date of publication of application:
**17.06.2009 Bulletin 2009/25**

(73) Proprietor: **Sandler AG**
**95126 Schwarzenbach/Saale (DE)**

(72) Inventors:
• **JAEGER, Jobst, T.**
**41453 Neuss (DE)**
• **JUNG, Dieter**
**41453 Neuss (DE)**
• **BERNHUBER, Uwe**
**95032 Hof/Saale (DE)**

• **HÖFLICH, Wolfgang**
**95126 Schwarzenbach (DE)**
• **TESCH, Walter**
**41453 Neuss (DE)**

(74) Representative: **Wilhelm, Stefan et al**
**3M Deutschland GmbH**
**Office of Intellectual**
**Property Counsel**
**Carl-Schurz-Strasse 1**
**41453 Neuss (DE)**

(56) References cited:
EP-A1- 0 820 747          WO-A1-96/16216
US-A- 5 167 897          US-A- 5 328 758
US-A- 5 501 679          US-A- 5 733 635
US-A- 5 804 286          US-A- 6 159 584
US-A1- 2004 191 470     US-B1- 6 506 698
US-B2- 7 078 089          US-B2- 7 078 089

**Description**

Field of the invention

The present invention relates to an activatable zero strain composite laminate comprising an elastic laminate having an elastic core layer and at least one skin layer, and at least one nonwoven layer which is attached to such elastic laminate. The present invention furthermore relates to an activated composite laminate which is obtainable by stretching said activatable zero strain composite laminate, and to a method of preparing said activated composite laminate.

Background of the invention

US 5,501,679 discloses activated microtextured elastomeric laminates comprising at least one elastomeric core layer and at least one thin skin layer. The activated microstructured elastomeric laminate is preferably prepared by coextrusion of the core and skin layer followed by stretching the laminate past the elastic limit of the skin layers and then allowing the laminate to recover. It is disclosed in US '679 that the shrink recovery mechanism of the laminate can be influenced by varying the thickness of the skin layer or layers. It is furthermore disclosed in US '679 that the texture of the microstructured laminate can be influenced by varying the manner in which the film is stretched past its elastic limit. It is disclosed that the laminate can be stretched uniaxially, sequentially biaxially or simultaneously biaxially. US '679 discloses various uses and applications for the stretched microstructured laminate including its use in diapers and garments.

Activatable composite laminate webs having an elastic core layer and at least one skin layer which is less elastic than the core layer are described, for example, in EP 0,521,883. In such laminate, the at least one skin layer and the at least one core layer form preferential activation regions and non-preferential activation regions wherein said at least one core layer is substantially elastomeric in at least said preferential activation regions. The at least one skin layer and/or the at least one core layer are provided such that when the multilayer laminate is stretched, said preferential regions can elongate and recover, in the elongated regions, to an elastic state. Nonwoven materials such as, for example, garments can be applied to the activatable or activated laminate, respectively, by ultrasonic welding, heat sealing and adhesives.

US 6,159,584 discloses an extensible elastic tab designed to be adhered to the edge of an article, comprising a coextruded elastic film with at least one elastic layer and at least one second layer on at least a first face of the elastic layer with at least one face of the coextruded elastic film attached to at least a partially extensible nonwoven layer. The partially expandable or extensible nonwoven layer has at least one first portion with limited extensibility in a first direction and at least one second inextensible portion in the first direction. The extensible elastic tab when stretched to the extension limit of the first portion or portions in the first direction will elastically recover at least 1.0 cm, preferably at least 2 cm providing an elastic tab having a Useful Stretch Ratio of at least 30 %. The Useful Stretch Ratio includes the portion of the elastic recovery length having an elastic recovery force of greater than 20 grams/cm force, but below a given extension which generally is 90 % of the extension limit. The extensible elastic tab of US '584 is disclosed to be particularly useful as a diaper fastening tab. The extensible non-woven layer is selected so as to allow the elastic film to stretch in the extensible portion of the nonwoven layer up to an expansion limit of this portion of the nonwoven layer. Beyond this expansion limit the incremental forces required to further stretch the extensible nonwoven layer portion increase generally by at least 100 grams/cm, preferably at least 200 grams/cm.

The laminates of EP 0,521,883 described above can be activated by a so-called post laminate-formation modulus or stress treatment in order to generate the required preferential activation regions and the non-preferential activation regions, respectively. Such treatment may include post formation annealing, selective cross-linking or selective plasticization. Post formation stress localization can be effected by localized corona treatment, mechanical ablation, scoring, cutting out laminate material, indentation or controlled localized stretching.

The controlled localized stretching treatment method is described to some detail in US 5,167,897. US '897 refers to a zero strain stretch laminate which comprises a first ply of a stretchable and elastomeric material and a second ply comprising an elongatable but not necessarily elastomeric material such as, for example, a nonwoven material. The two plies are attached to each other while in a substantially untensioned ("zero strain") condition. The zero strain stretch laminate is fed into a pair of opposed pressure applicators to effect incrementally stretching while subjecting opposed peripheral edge portions of said laminate web to restraint. The second ply will, upon stretching of the laminate, be at least to a degree permanently elongated so that, upon release of the applied tensile forces, it will not return to its original undistorted configuration. Depending on the properties of the second ply material and the degree of incremental stretching, the second ply material may undergo local Z-direction rupturing or Z-direction bulking, respectively, resulting in subsequent elastic extensibility in the direction of initially stretching.

Zero strain composite laminates comprising an elastic laminate and at least one nonwoven layer attached to it, may exhibit when activated by means of the method disclosed in US '897, locally distorted and/or damaged nonwoven layers which is not always desirable from an aesthetical point of view.

US 7,078,089 discloses an elastic laminate material comprising extensible nonwoven fibrous webs attached to a thermoplastic elastic material. The nonwoven fibrous webs may be stable fiber webs which are not bonded until such time as they are laminated to the thermoplastic elastic material. This means in other words that the bonding which is typically effected by thermal print bonding, both solidifies the non-bonded nonwoven fibrous web and attaches it to the thermoplastic elastic material. The thermoplastic elastic material may be a single-layer or multi-layer film. Multi-layer films comprise one or two external skin layers which comprise a bonding agent to effect bonding of the elastic to the nonwoven fibrous web.

It was found that the nonwoven fibrous web layer(s) of the elastic laminate of US '089 tend to rupture between the bonding points upon activation which results in an aesthetical less attractive appearance of the elastic laminate material.

WO 96/16,216 provides a composite nonwoven fabric with a superior combination of extensibility, tensile properties and abrasion resistance. The composite nonwoven fabric comprises at least one layer containing multipolymer fibers, with a plurality of bonds bonding the fibers together to form a coherent extensible nonwoven web. The coherent extensible nonwoven web has a Taber surface abrasion value (rubber wheel) of greater than 10 cycles and an elongation at peak load in at least one of the machine direction or the cross-machine direction of at least 70 percent. A second extensible layer is laminated to this coherent extensible nonwoven web by an adhesive layer. While the use of multicomponent fibers may improve the Taber surface abrasion value, it does not avoid damaging and/or rupturing of the nonwoven layers during activation to a sufficient extent.

It was therefore an object of the present invention to provide an activatable zero strain composite laminate comprising one or more nonwoven layers which can be activated to the corresponding activated composite laminate essentially without damaging and/or rupturing the nonwoven layers while simultaneously providing acceptable and/or advantageous elastic properties of the activated composite laminate. It is another object of the present invention to provide a method capable of activating said activatable zero strain skin layers.

composite laminate essentially without damaging and/or rupturing the nonwoven layer. Other objects of the present invention will be evident for the person skilled in the art from the detailed description of the invention below.

<u>Brief description of the invention</u>

The present invention relates to an activatable zero strain composite laminate web comprising an activatable elastic laminate web having an elastic core layer and at least one skin layer which is less elastic than the core layer, and at least one pre-bonded staple fiber nonwoven web which is attached to one of the skin layers of the elastic laminate web, the at least one staple fiber nonwoven web having an elongation at break of at least 100 % in the cross-direction and said activatable elastic laminate web forming an essentially homogeneous microtextured surface when stretched in the first upload in the cross-direction past the elastic limit of the one or more skin layers.

The present invention furthermore relates to an activatable zero strain composite laminate web comprising an activatable elastic laminate web having an elastic core layer and at least one skin layer which is less elastic than the core layer, and at least one pre-bonded staple fiber nonwoven web which is attached to one of the skin layers of the elastic laminate web, the at least one staple fiber nonwoven web having an elongation at break of at least 100 % in the cross-direction and a bonding area of between 8 and 22 %, more preferably of between 9 and 18 % and especially preferably of between 9 and 15 % with respect to the surface of the non-woven web, said activatable elastic laminate web having a ratio of the thickness of the core layer of the elastic laminate web over the thickness of the at least one skin layer or the sum of the skin layers, respectively, of such web of at least 6:1 wherein at least one staple fiber nonwoven layer is attached to the elastic laminate by a discontinuous adhesive layer.

The present invention furthermore relates to an activated composite laminate which is obtainable by stretching said activatable composite laminate of the present invention in the cross-direction past the elastic limit of the one or more skin layers but below the elongation at break of the pre-bonded staple fiber nonwoven web.

The present invention furthermore relates to a method of preparing an activatable composite laminate according to the invention comprising the steps of

(i) providing an activatable elastic laminate web having an elastic core layer and at least one skin layer which is less elastic than the core layer, said activatable elastic laminate web forming an essentially homogeneous microtextured surface when being stretched in the first upload in cross-direction past the elastic limit of the one or more skin layers;
(ii) providing at least one pre-bonded staple nonwoven web having an elongation at break of at least 100 % in cross-direction; and
(iii) attaching said stable nonwoven web to said elastic laminate web whereby the activatable elastic laminate web is held in an essentially untensioned state.

The present invention furthermore comprises a method of preparing an activated composite laminate web according to the invention comprising the steps of

(i) providing an activatable composite laminate web according to this invention; and
(ii) stretching such activatable composite laminate web in the cross-direction past the elastic limit of the one or more skin layers but below the elongation at break of the pre-bonded staple fiber nonwoven layer web.

Brief description of the figures

*Fig. 1* is a schematic cross-sectional view of a portion of an activatable zero strain composite laminate web 10 of the present invention comprising an activatable elastic laminate web having an elastic core layer 1 and a skin layer 2. The activatable composite laminate web 10 further comprises a pre-bonded staple fiber nonwoven web 4 which is attached to the skin layer 2 via adhesive layer 3.

*Fig. 1a* is a schematic cross-sectional view of a portion of another activatable zero strain composite laminate web 10 of the present invention which differs from the embodiment of Fig. 1 in that the elastic laminate web comprises a second skin layer 3 bonding a second pre-bonded staple fiber nonwoven web 4.

*Fig. 1b* is a schematic cross-sectional view of a portion of another activatable zero strain composite laminate web 10 of the present invention which differs from the embodiment of Fig. 1 in that it comprises a discontinuous adhesive layer 3 comprising adhesive strips 3a.

*Fig. 2* is a schematic cross-sectional view of a portion of an activated composite laminate web 20 of the present invention which is obtainable by stretching the activatable composite laminate 10 of Fig. 1a in the cross-direction.

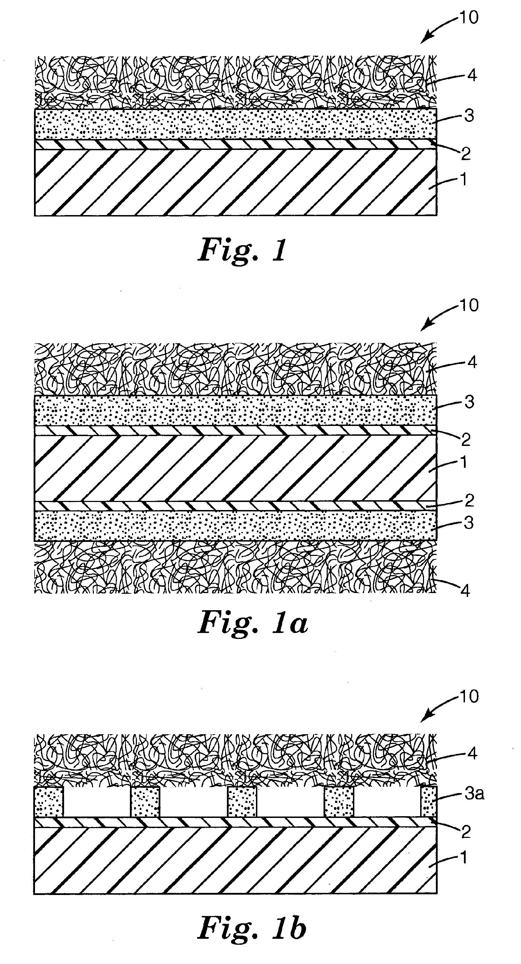

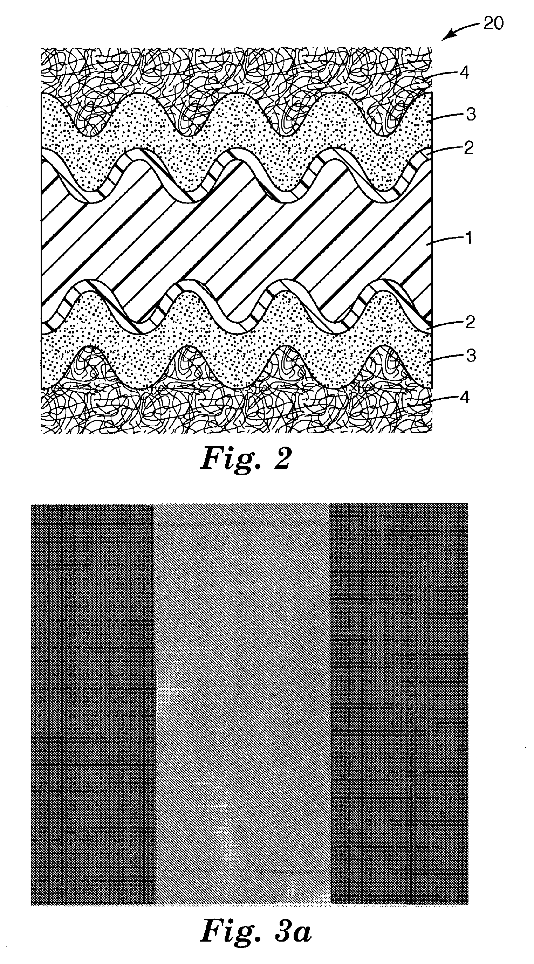

Figs. 1, 1a, 1b and 2 are highly schematic and do not reproduce the thickness of the various layers in proportion so that no dimensions may be taken from said Figs. In said Figs. the nonwoven layers are not shown in their full extension but they are cut off.

*Fig. 3a* shows a photograph of a portion of an activated elastic laminate web useful in the present invention, which was stretched in the cross-direction (i. e. along the length of the portion) to an elongation of 40 % (first upload) and then relaxed. The photograph shows the stretched elastic laminate in the relaxed state obtained after the first unload. The portion of the elastic laminate web shows a homogeneous opaque appearance. The composition and geometrical dimension of the elastic laminate web is described in Example 1.

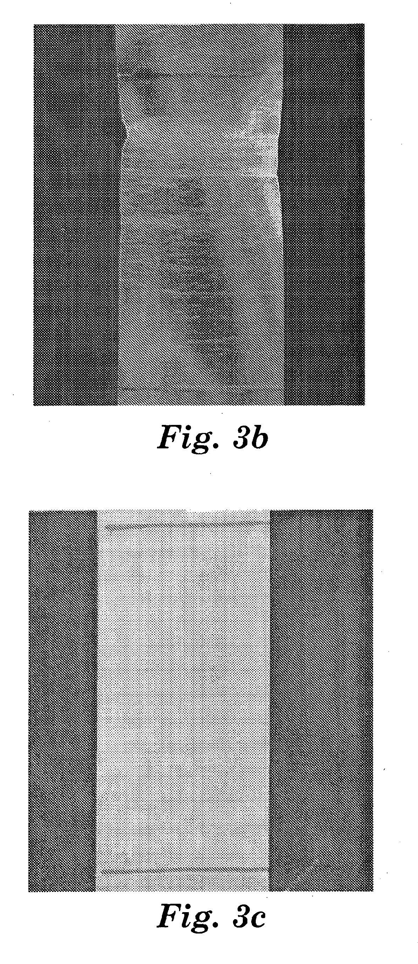

*Fig. 3b* shows a photograph of a portion of an activated elastic laminate web not useful in the present invention, which was stretched in the cross-direction (i. e. along the length of the portion) in the first upload to an elongation of 40 % and then relaxed. The photograph shows the activated elastic laminate in the relaxed state subsequent to the first unload. The portion of the elastic laminate web shows an inhomogeneous appearance showing a necked-in, milky section in the upper half of the web portion and an essentially clear appearance in the lower half of the web portion. The composition and geometrical dimensions of the elastic laminate web is described in Comparative Example 1.

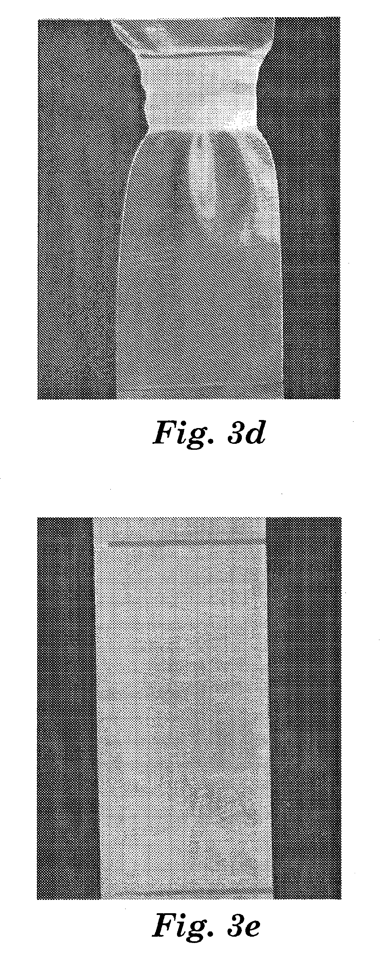

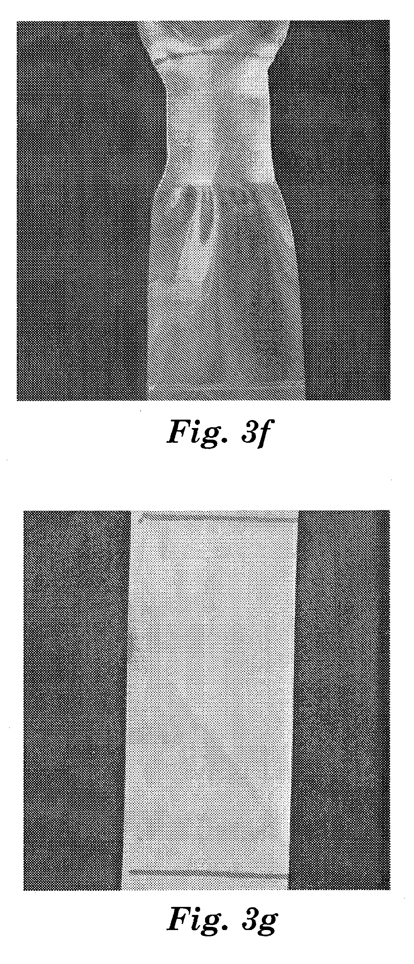

*Figs. 3c, 3e and 3g* show photographs of the portion of the activated elastic laminate web of Fig. 3a useful in the present invention, in a relaxed state after the first unload subsequent to a first upload to an extension of 80 %, 120 % and 160 %, respectively.

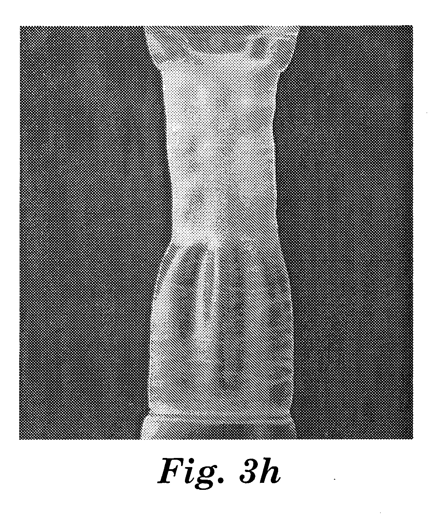

*Figs. 3d, 3f and 3h* show photographs of the portion of the activated elastic laminate web of Fig. 3b not useful in the present invention, in a relaxed state after the first unload subsequent to a first upload to an extension of 80 %, 120 % and 160 %, respectively.

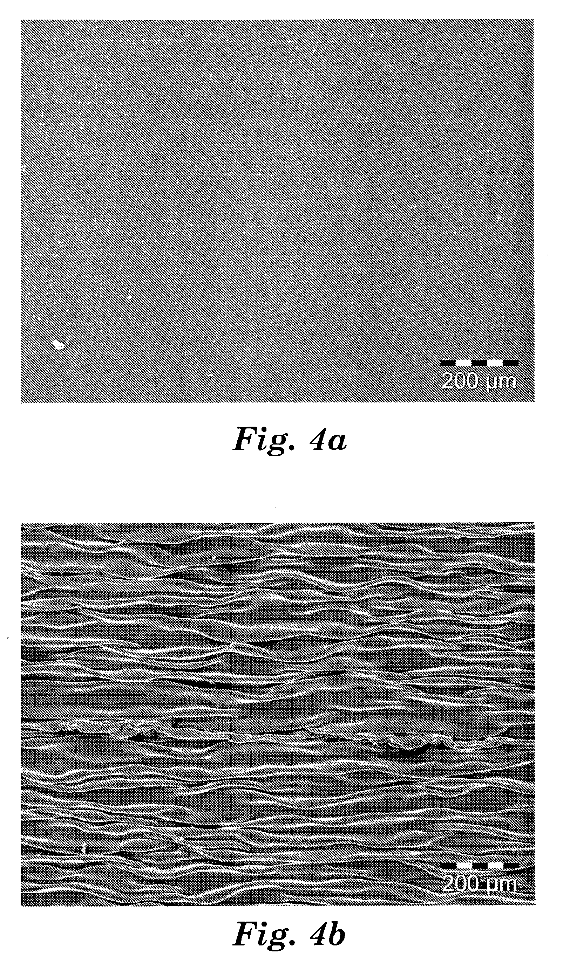

*Fig 4a* shows a microphotograph of the surface of the portion of the elastic laminate web of Fig. 3d which was taken at a magnification of 100x in the clear section in the lower half of such web portion.

*Fig 4b* shows a microphotograph of the surface of the portion of the elastic laminate web of Fig. 3d which was taken at a magnification of 100x in the opaque necked-in section of such web portion.

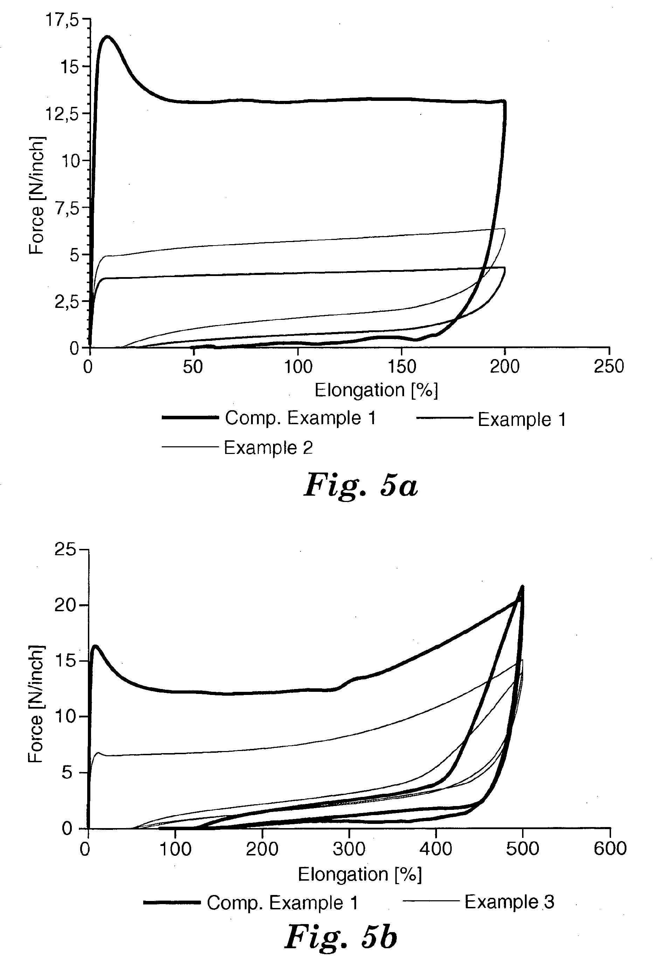

*Fig. 5a* shows a series of stress-strain curves for different elastic laminate webs comprising in each case the first upload and the first unload, as follows:

> ___: elastic laminate web of Example 1
> ___: elastic laminate web of Example 2
> ___: elastic laminate web of Comparative Example I

*Fig. 5b* shows a series of stress-strain curves for two different elastic laminates comprising for each laminate the first upload and unload and the fifth upload and unload, as follows:

> ___: elastic laminate web of Example 3
> ___: elastic laminate web of Comparative Example 1

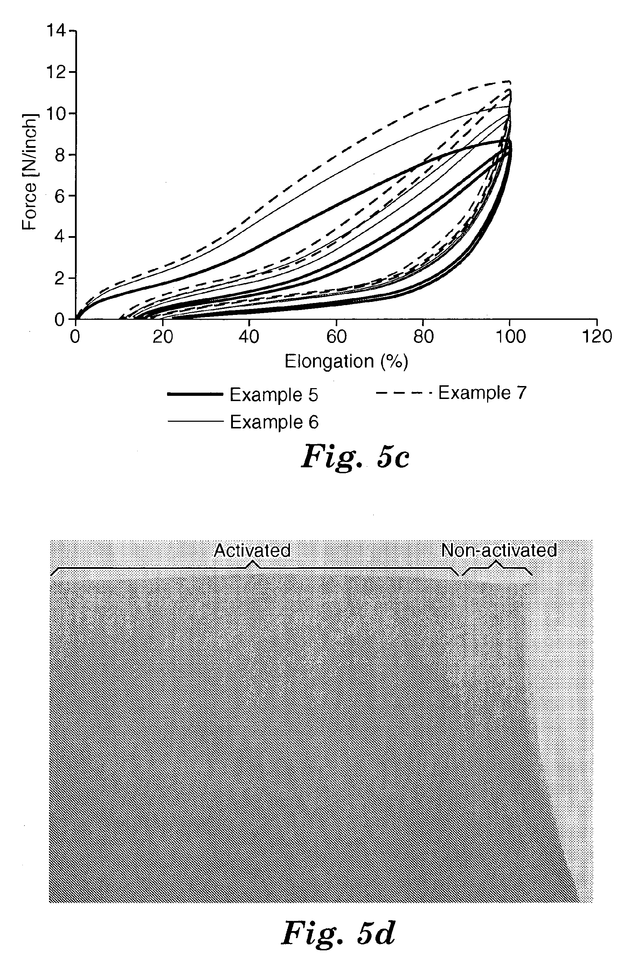

*Fig.* 5c shows a series of stress-strain curves for three different composite laminate webs of the present invention comprising for each composite laminate web the first upload and unload, the second upload and unload and the third upload and unload, respectively, as follows:

> ___: composite laminate web of Example 5
> ___: composite laminate web of Example 6
> ___: composite laminate web of Example 7

The composite laminate web of Example 5 comprises the elastic web laminate of Example 1; the composite laminate web of Example 6 comprises the elastic web laminate of Example 2; and the composite web laminate of Example 7 comprises the elastic web laminate of Example 3.

Fig. 5d shows a photograph of the composite laminate web of Example 6 where a strip section of the right edge of the composite laminate web extending in the machine direction is activatable (i. e. non-activated) whereas all the rest of the composite laminate web shown in Fig. 5 a is activated.

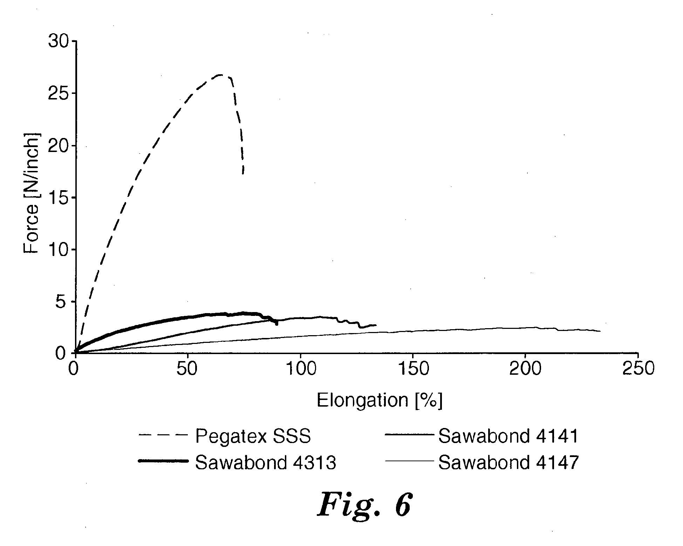

*Fig.* 6 shows a series of stress-strain curves for various nonwoven webs as follows:

> ----- : Pegatex SSS (spunbond nonwoven web)
> ___ : Sawabond 4313 (carded nonwoven web)
> ___ : Sawabond 4141 (carded nonwoven web)
> ___: Sawabond 4147 (carded nonwoven web)

Detailed description of the invention

The term activatable "zero strain" composite laminate as used above and below relates to a laminate comprising an elastic laminate having an elastic core layer and at least one skin layer which is less elastic than the core layer, and at least one pre-bonded staple fiber nonwoven layer. The elastic laminate and the pre-bonded staple fiber nonwoven layer are attached to each other by securing the nonwoven layer, either intermittently or continuously, to one of the skin layers while the elastic laminate is in an essentially unstretched ("zero strain") condition. The terms "activatable" and "non-activated" are used synonymously. If it is clear from the context whether the elastic laminate web or the composite laminate web, respectively, are activatable or activated, such terms may also be omitted.

The composite laminate is designated as a web if reference is made to a laminate extending over a significant length in the machine direction. Smaller sections of such composite laminate web which are usually obtained by cutting the web in the cross-direction, are referred to as composite laminate portions.

Above and below, the composite laminate web and/or portions of the composite laminate web are also simply referred to as composite laminate.

Above and below, the elastic laminate web and/or portions of such elastic laminate web are also simply referred to as elastic laminate.

The term "machine direction" (MD) as used above and below denotes the direction of the running, continuous web of the elastic laminate web or the composite laminate, respectively. The term "cross-direction" (CD) as used above and below denotes the direction which is essentially normal to the machine direction. If portions of the elastic laminate web or the composite laminate web, respectively, are used, the terms CD and MD as defined above and below are used to characterize the orientation of such portions as well.

The elastic core layer of the activatable zero strain composite laminate is formed of a material, which exhibits elastomeric properties at ambient conditions. Elastomeric means that the material will substantially resume its original shape after being stretched. Preferably, the elastomer will sustain only small permanent set following deformation and relaxation, which set is preferably less than 30 % and more preferably less than 20 % of the original 50 to 500 % stretch. The elastomeric material can be either pure elastomers or blends with an elastomeric phase or content that will still exhibit substantial elastomeric properties at room temperature. Suitable elastomeric thermoplastic polymers include block copolymers such as those known to those skilled in the art as A-B or A-B-A type block copolymers or the like. These block copolymers are described, for example, in US patents 3,265,765; 3,562, 356; 3,700,633; 4,116,917 and 4,156,673, the substance of which are incorporated herein by reference. Styrene/isoprene, butadiene or ethylene-butylene-styrene (SIS, SBS or SEBS) block copolymers are particularly useful. Generally, there are two or more blocks, at least one A-block and at least one B-block, where the blocks can be arranged in any order including linear, radial, branched, or star block copolymers. Other useful elastomeric compositions can include elastomeric polyurethanes, ethylene copolymers such as ethylene vinyl acetates, ethylene/propylene copolymer elastomers or ethylene/propylene diene copolymer elastomers. Blends of these elastomers with each other or with modifying non-elastomers are also contemplated.

Viscosity reducing polymers and plasticizers can also be blended with the elastomers such as low molecular weight polyethylene and polypropylene polymers and copolymers, or tackifying resins such as Wingtack™, aliphatic hydrocarbon tackifiers available from Goodyear Chemical Company. Tackifiers can also be used to increase the adhesiveness of an elastomeric layer to a skin layer. Examples of tackifiers include aliphatic or aromatic hydrocarbon liquid tackifiers, polyterpene resin tackifiers, and hydrogenated tackifying resins. Aliphatic hydrocarbon resins are preferred.

Additives such as dyes, pigments, antioxidants, antistatic agents, bonding aids, antiblocking agents, slip agents, heat stabilizers, photo stabilizers, foaming agents, glass bubbles, reinforcing fiber, starch and metal salts for degradability or microfibers can also be used in the elastomeric core layer(s).

The skin layers of the activatable zero strain composite laminate generally comprise non-tacky materials or blends formed of any semi-crystalline or amorphous polymer(s) which are less elastomeric than the elastic core layer. The skin layers are preferably essentially inelastic and non-tacky, and thus will undergo relatively more permanent deformation than the core layer at the percentage that the elastic laminate is stretched. Elastomeric materials such as olefinic elastomers, e. g., ethylene-propylene elastomers, ethylene propylene diene polymer elastomers, metallocene polyolefin elastomers or ethylene vinyl acetate elastomers, or styrene/isoprene, butadiene or ethylene-butylene/styrene (SIS, SBS or SEBS) block copolymers, or polyurethanes or blends with these materials can be used as long as the skin layers provided are essentially non-tacky and less elastomeric than the core layer. The skin layers preferably can act as barrier layers to any adhesive applied. The elastomeric materials used are present in a blend with non-elastomeric materials in a weight percent range of 0 to 70 %, preferably 0 to 50 % and more preferably 0 to 20 %. High percentages of elastomer in the skin layer(s) generally require use of antiblock and/or slip agents to reduce the surface tack and roll unwind force. Preferably, the skin layers comprise one or more polyolefin or polyolefin copolymer selected from the group consisting of polyethylene, polypropylene, polybutylene, and polyethylene-polypropylene copolymer. The skin layers, however, may also include one or more polyamides such as nylon, one or more polyesters such as polyethylene terephthalate, and suitable blends thereof.

It is essential in the present invention to control the core:skin thickness ratio and/or the softness of the skin layer(s) to allow for an essentially homogeneous activation of the activatable elastic laminate. The core:skin thickness ratio as used above and below is defined as the ratio of the thickness of the elastic core over the thickness of the at least one skin layer (if only one skin layer is present) or over the sum of the thicknesses of the two skin layers (if a second skin layer is present), respectively.

It was found that if the core:skin thickness ratio defined above is too low and/or the skin layers are too rigid the activatable elastic laminate when stretched, tends to neck macroscopically and/or form macroscopic buckles. The term "macroscopic(ally)" as used above and below means that such necked-in sections and/or buckles can be easily seen with the unaided eye. Typically the necked-in sections or the buckles have an extension of at least I mm.

These macroscopic neckings or buckles often only form in certain areas of the stretched elastic laminate whereas other areas of the stretched elastic laminate where the skin layers are essentially not distorted, remain flat and/or non-necked. This inhomogeneous activation behaviour of the elastic laminate imparts an unfavourable aesthetic appearance to the activated composite laminate which is not acceptable, in particular, for use in hygienic articles such as diapers. Also, the inhomogeneous activation behaviour results in a stress-strain behaviour which may be difficult to control. Once the necking and/or buckling area or areas of the elastic laminate have been stretched to an extent so that the force applied may be sufficient to induce necking and/or buckling in other area(s), the stress-elongation curve may exhibit further peaks, and further stretching of the elastic laminate may be zippy.

The core:skin thickness ratio and/or the softness of the skin layers of the elastic laminate need to be selected so that the skin layer(s) when stretched beyond their elastic limit and relaxed with the core form a microstructured surface texture. Microstructure means that the surfaces of the skin layers of the elastic laminate contain microscopic peak and valley irregularities, folds or other microscopic surface structure elements which are large enough to be perceived by the unaided human eye as causing increased opacity over the opacity of the elastic laminate before microstructuring, and which irregularities are small enough to be perceived as smooth or soft to human skin. Magnification of the irregularities is required to see the details of the microstructured texture.

The core:skin thickness ratio and/or the softness of the skin layers of the elastic laminate furthermore needs to be selected so that the elastic laminate can be stretched essentially homogeneously as indicated by the absence of any macroscopic buckles and/or an essentially homogeneously increased opacity of the elastic laminate as compared to the initial opacity before stretching the elastic laminate.

The activation behaviour of an activatable elastic laminate can be easily assessed qualitatively as follows. An essentially clear, activatable elastic laminate web which exhibits a favourable activation behaviour and is suitable for use in the present invention is rendered essentially homogeneously opaque when stretching a portion of such laminate to a relatively low extension of, for example, 40 - 160 % in the cross-direction with subsequent relaxation. Stretch-relaxing of an initially opaque activatable elastic laminate renders the activated elastic laminate more opaque in comparison to the non-activated elastic laminate.

This behaviour is exemplified in Fig. 3a, 3c, 3e and 3g for the essentially clear activatable elastic laminate of Example 1 below having a core:skin thickness ratio of about 8.2:1.

Fig. 3a is a photograph of a portion of such elastic laminate after stretching it to an elongation of 40 % in the cross-direction with subsequent relaxation. It can be seen that the activated elastic laminate exhibits a homogeneous opaque appearance.

The photographs of Figs. 3c, 3e and 3g show the surface of the activatable elastic laminate of Example 1 upon stretch-relaxing it to elongations of 80 %, 120 % and 160 %, respectively. A visual comparison of Figs. 3a, 3c, 3e and 3g shows that the opacity visibly increases from Fig. 3a to 3c when increasing the elongation from 40 % to 80 % whereas no distinct increase in opacity can be visually detected when stretch-relaxing to elongations of 120 % or 160 %, respectively.

The activation behaviour of an elastic laminate web which is not suitable for use in the present invention is exemplified in contrast by Figs. 3b, 3d, 3f and 3h which show the surface of portions of the elastic laminate web of Comparative Example 1 upon stretching them to elongations of 40 %, 80 %, 120 % and 160 % and subsequent relaxing. The essentially clear activatable elastic laminate of Comparative Example 1 below has a core:skin thickness ratio of about 4.5:1. Fig. 3b shows that the activation of the elastic laminate portion begins in an area in the upper half of such portion which has been rendered slightly opaque and shows a slight necking. The other areas of the elastic laminate portion of Fig. 3b are not affected and remain essentially clear.

It can be taken from Figs. 3d, 3f and 3h that upon stretch-relaxing the elastic film portions to higher elongations of 80 %, 120 % and 160 %, the activated area in the upper half of the elastic laminate portion is getting larger and more opaque while the areas in the lower half of the elastic laminate portion remain essentially clear. The elastic film laminate portions of Figs. 3d, 3f and 3h show a pronounced necking in the activated area in the upper half of such portion and the formation of macroscopic buckles, in particular, in the transition area between the activated upper half and the non-activated lower half of the elastic laminate portion.

The core:skin layer thickness ratio of elastic laminates suitable in the present invention preferably is at least 6:1, more preferably at least 7:1 but less than 1,000:1 and most preferably between 7:1 and 25:1.

The thickness of the core layer preferably is between 20 and 240 $\mu$m and more preferably between 40 and 150 $\mu$m. The thickness of the skin layer preferably is between 1 and 10 $\mu$m and more preferably between 2 and 8 $\mu$m. The overall thickness of the elastic laminate preferably is between 25 and 250 $\mu$m.

While the addition of the skin layers to the core layer generally tends to reinforce the activatable elastic laminate of the present invention, the skin layers are provided to be sufficiently thin and/or soft so that little or no reinforcement of the activated elastic laminate occurs, and that the activated elastic laminate is elastic in its initial elongation as well as in its second and subsequent elongations at suitable low stress elongation forces and low hysteresis loss levels when the elastic is cycled in use (e. g. in hygienic disposable products by dimensional changes caused by the wearer's breathing). Preferably, the elastic laminate does not have a distinct yield point or range in the first activation cycle. In the subsequent cycles the elastic properties of the elastic laminate preferably are similar to and/or tend to approach the elastic properties of the elastic core itself.

The activation behaviour of elastic laminates can furthermore be assessed by recording the first stress-elongation cycle of the activatable elastic laminate.

Fig. 5a shows, for example, the first activation cycle for the activatable elastic laminates of Example 1 (-), Example 2 (-) and Comparative Example I (-), respectively.

The first upload curves for all three elastic laminates of Example 1, Example 2 and Comparative Example 1 initially show a steep increase in the stress-elongation curve until the force required to impart a microstructure to the skin layers of the elastic laminate has been reached. The elastic laminates of Examples 1 and 2 which are suitable in the present invention do not show, however, a distinct peak or yield point in the stress-elongation curve. This is in sharp contrast to the activation behaviour of the elastic laminate of Comparative Example 1 which is not suitable for use in the present invention. The elastic laminate of Comparative Example 1 exhibits a pronounced initial yield point which is related to an initial deformation and/or distortion of the skin layers. The force required to further elongate the elastic laminate of Comparative Example 1 drops to a distinctly lower level as compared to the peak force value related to the initial deformation and/or distortion of the skin layers.

The elastic laminates which are useful in the present invention preferably exhibit in the first upload conducted at a speed of 127 mm/min

- essentially no initial yield point related to an initial deformation and/or distortion of the skin layers as is exemplified by the elastic laminate of Example I, or
- if such initial yield point is present, a ratio of the force at such initial peak or yield point over the force at an elongation of 50 % of less than 1.15, more preferably of less than 1.10 and especially preferably of less than 1.05.

Preferably, the elastic laminate exhibits when stretched in the first upload to an elongation of 200 % a retraction force in the first unload at an elongation of 80 % of at least 0.3 N/inch and more preferably of at least 0.5 N/25.4 mm.

The activatable zero strain composite material preferably comprises one or two nonwoven layers which are attached to the exposed surfaces of the one or two skin layers, respectively. The nonwoven layers used in the present invention are made from pre-bonded staple fiber webs having an elongation at break in cross-direction as measured according to the method specified in the test section below of at least 100 %, more preferably of at least 120 % and especially preferably of at least 150 %. The pre-bonded staple fiber webs suitable in the present invention include airlaid, wet-laid and carded nonwoven webs with carded nonwoven webs being preferred.

Carded non-woven webs are made from separated staple fibers which fibers are sent through a combing or carding unit which separates and aligns the staple fibers in the machine direction so as to form a generally machine direction-oriented fibrous nonwoven web. If desired, the degree of machine direction orientation may be reduced and/or adjusted by randomizers.

Once the carded web has been formed, it is then bonded by one or more of several bonding methods. One bonding method is powder bonding wherein a powdered adhesive is distributed through the web and then activated, usually by heating the web and adhesive with hot air. Another bonding method is pattern bonding wherein heated calendar rolls or ultrasonic bonding equipment are used to bond the fibers together.

The pre-bonded staple fiber webs useful in the present invention preferably exhibit a localized discontinuous bond pattern such as, for example, a multiplicity of discrete thermal bond points throughout the web.

It is essential that the staple fiber webs used in the present invention are pre-bonded. Non-pre-bonded carded nonwoven webs which are used in US 7,078,089 can only be processed in-line. Contrary to this it is preferred in the present invention to process the nonwoven web and the elastic laminate web separately which requires imparting integrity to the nonwoven web by pre-bonding. Non-pre-bonded nonwoven webs furthermore tend to form a fuzzy or curly surface upon stretch activation because the ends of unbonded individual fibers may stick out of the surface. This is not acceptable, in particular, for baby hygiene products such as baby diapers where the baby may try to rip off such lose fibers and may swallow them.

In the composite laminate webs of the present invention the pre-bonded nonwoven webs are attached to the elastic laminate by a separate bonding mechanism such as by thermo bonding, ultrasonic bonding or, preferably, adhesive bonding. The pre-bonded bonding pattern of the nonwoven web is therefore separate from and independent of the bonding mechanism between the nonwoven web and the elastic laminate web. Although the present inventors do not wish to be bound by such explanation it is speculated that such independent and separate pre-bonding pattern of the nonwoven web provides a multiplicity of joints which imparts on the one hand a favourable stretching behaviour and a high elongation at break to the nonwoven web while maintaining on the other hand a sufficient integrity of the nonwoven web.

The pre-bonded nonwoven webs suitable in the present invention preferably exhibit a bonding area of at least 8 %, more preferably of at least 9 % and especially preferably of at least 9.5 % with respect to the surface of the nonwoven web. If the bonding area of the nonwoven web is less than 8 % with respect to the surface of the nonwoven web the mechanical integrity of such web tends to be insufficient, in particular, for hygienic applications.

The pre-bonded staple fiber nonwoven webs of the present invention are anisotropic because they are distinctly stronger in the machine direction in which the fibers are oriented by the combing or carding step as compared to the cross-direction. Generally, with increasing bonding area of the pre-bonded nonwoven web, its tensile strength in the cross-direction will increase and the elongation at break will decrease. In the present invention, the bonding area of the pre-bonded nonwoven web and the average degree of orientation of the fibers in the machine direction (which can be adjusted by using randomizers as was described above) are selected so that the ratio of the tensile strength at break in the machine direction over the tensile strength of break in the cross-direction is preferably between about 5:1 to 7:1 and more preferably between about 5.3:1 to 6.7:1.

It was furthermore found that the pre-bonded nonwoven webs useful in the present invention preferably exhibit a bonding area of not more than 22 %, more preferably of less than 18 % and especially preferably of less than 15 % with respect to the surface of the nonwoven web. If the bonding area of the pre-bonded nonwoven web is higher than 22 % with respect to its surface, the nonwoven web tends to be too strong in the cross-direction and the elongation at break in the cross-direction tends to be too low.

The bonding area of the pre-bonded nonwoven web preferably is between 9 and 18 % and more preferably between 9 and 15 % with respect to the surface of the nonwoven web.

The staple fiber nonwoven webs suitable in the present invention preferably comprise one or more fibers selected from the group consisting of natural or synthetic fibers selected from cotton, rayon, polyolefins including polyethylene and polypropylene, polyamides including nylon, polyesters including polyethylene terephthalate, aramids and blends thereof. Polyolefin based fibers are generally preferred.

The staple fiber nonwoven webs suitable in the present invention preferably have an average staple length in the machine direction of between 30 and 80 mm and more preferably of between 30 and 60 mm.

The activatable zero strain composite laminate of the present invention is preferably obtained by bonding the nonwoven layer(s) to the skin layer(s) of the elastic laminate with both the elastic laminate and the nonwoven layer(s) being essentially in a non-tensioned ("zero strain") state. With respect to the elastic laminate the term "non-tensioned" or "zero strain" means that the elastic laminate is kept in an unelongated state where it does essentially not exhibit an elastic retraction force. With respect to the nonwoven layer(s) the term "non-tensioned" or "zero strain" means that the nonwoven is present in an essentially non-corrugated, flat form. In order to establish "zero strain" lamination conditions, the winding tension is preferably kept at a low level, preferably 50 N/m or less. Further details on "zero strain" lamination conditions can be taken, for example, from US 6,476,289, US 5,422,172 and US 5,167,897. The substance of the corresponding parts of such references is incorporated herein by reference.

Bonding can be effected, for example, by heat bonding, extrusion bonding or, preferably, adhesion bonding.

Preferred adhesives are those activatable by pressure, heat or combination thereof. Suitable adhesives include those based on acrylate, rubber resin, epoxies, urethanes or combinations thereof. The adhesive layer may be applied by solution, water-based or hot-melt coating methods. The adhesive can include hot-melt coated formulations, as well as laminating, thermally-activated, and water-activated adhesives and bonding agents. Useful adhesives according to the present invention include all pressure-sensitive adhesives.

Pressure-sensitive adhesives are well known to possess properties including: aggressive and permanent tack, adherence with no more than finger pressure, and sufficient ability to hold onto an adherent. Examples of adhesives useful in the invention include those based on general compositions of polyacrylate; polyvinyl ether; diene rubber such as natural rubber, polyisoprene, and polybutadiene; polyisobutylene; polychloroprene; butyl rubber; butadiene-acrylonitrile polymer; thermoplastic elastomer; block copolymers such as styrene-isoprene and styrene-isoprene-styrene (SIS) block copolymers, ethylene-propylene-diene polymers, and styrene-butadiene polymers; poly-alpha-olefin; amorphous polyolefin; silicone; ethylene-containing copolymer such as ethylene vinyl acetate, ethylacrylate, and ethyl methacrylate; polyurethane; polyamide; epoxy, polyvinylpyrrolidone and vinylpyrrolidone copolymers; polyesters; and mixtures or blends (continuous or discontinuous phases) of the above. Additionally, the adhesives can contain additives such as tackifiers, plasticizers, fillers, antioxidants, stabilizers, pigments, diffusing materials, curatives, fibers, filaments, and solvents. Also the adhesive optionally can be cured by any known method.

Pressure-sensitive adhesives including hotmelt pressure-sensitive adhesives are preferred in the present invention because of their general viscoelastic properties which translate into favourable stretching properties.

Adhesion bonding can be effected by continuous or discontinuous adhesive layers, respectively.

While continuous adhesive layers tend to securely attach the nonwoven web layer to the skin layer of the elastic laminate web it was found by the present inventors that discontinuous adhesion patterns can provide composite laminate webs with unique properties.

The discontinuous adhesive patterns may be regular or irregular and comprise, for example, adhesive strips which exhibit an essentially straight zigzag or generally curved shape including, for example, a wavy shape whereby such strips preferably extend in the machine direction. The discontinuous adhesion pattern may also be applied, for example, by screen-printing or by brush-coating.

The bonding area of the discontinuous adhesive area preferably is between 10 and 95 % and more preferably between 20 and 90 % with respect to the surface are of the skin layers.

Especially preferred are composite laminates wherein the one or two nonwoven webs are bonded to the elastic laminate web by one or two adhesive layers comprising essentially straight, zigzag or regularly curved adhesive strips extending essentially in the machine direction.

Upon stretch-activation in the cross-direction and subsequent relaxation, the composite laminates tend to exhibit small ruffles with an extension in the $\mu$m or mm range in the adhesive-free space between adjacent adhesive strips which tends to provide a more fluffy and thicker appearance to the composite laminates. The width of the adhesive strips and of the adhesive-free space between adjacent adhesive strips needs to be selected so that the nonwoven web in the adhesive-free areas does not break and/or is not mechanically distorted to an unacceptable degree upon stretching the activatable composite laminate to a desired elongation of, for example, 100 to 150 %. It was found by the present inventors that these requirements are fulfilled, for example, by adhesive strips which exhibit a width in the cross-direction of preferably between 0.5 and 3 mm and more preferably of between 0.75 mm and 2.75 mm. The adhesive-free space between adjacent adhesive strips preferably is between 0.5 and 3 mm and more preferably between 0.75 and 2.75 mm.

The activatable zero strain composite laminate web of the present invention can be activated essentially without distorting and/or rupturing the nonwoven layer using, for example, the width stretching device disclosed, for example, in US 5,043,036.

Such device which is also referred to as diverging disks stretching device, comprises two circular pulleys or disks mounted on a frame for rotation about their axes with the axes being oriented to position portions of the peripheral surfaces of the pulleys at a close spacing at a first location relative to the frame, and to position portions of the peripheral surfaces of the pulleys at a far spacing significantly greater than the close spacing at a second location relative to the frame and diametrically across the pulleys from the first location. The device also comprises two continuous flexible belts. The belts and the pulleys have interacting guide means extending longitudinally along the belts and circumferentially around the peripheral surfaces of the pulleys for maintaining the belts in circumferential alignment around the peripheral surfaces of the pulleys. These interacting guide means are preferably provided by the peripheral surfaces of the pulleys having a plurality of spaced circumferentially extending ridges with recesses between the ridges, and the belts having along one side a plurality of longitudinally extending spaced ridges with recesses between the ridges. The ridges on the belts are adapted and aligned to enter the grooves in the pulleys, and the ridges on the pulleys are adapted and aligned to enter the grooves in the belts. The belt is mounted on the frame for movement along predetermined paths including clamping path portions with the interacting ridges and grooves on the belts and pulleys, respectively, in engagement from an inlet position adjacent said first location to an outlet position adjacent said second location with the belts being biased towards the pulleys.

In such device the two opposing edge areas of the activatable composite laminate web extending in the machine direction, are clamped at the inlet position to the peripheral surfaces of the pulleys by the belts, and the activatable composite laminate web is stretched to widen its width in the cross-direction as the pulleys rotate during the movement of the composite laminate web from the inlet position to the outlet position. The activated composite laminate being released from the interacting guide means at the outlet position of the device may be wound into a roll in its relaxed state.

The extent of cross-directional stretching provided by such diverging disks stretching device can be varied by varying the distance between the portions of the peripheral surfaces of the pulleys at the first and second location, respectively. If desired the extent of cross-directional stretching can be continuously varied over a wide range of, for example, between 10 and 500 %, more preferably between 20 and 300 % and especially preferably between 40 and 250 %.

The rate at which the activatable composite laminate is stretched can be varied by varying the rotation speed of the pulleys and/or the diameter of the pulleys. For a given rotation speed of the pulleys, larger diameter pulleys effect a slower rate of stretching than smaller diameter pulleys. If desired the stretching rate provided by the diverging disks stretching apparatus can be varied essentially continuously in a broad range of preferably from 10 m/min to 600 m/min. Preferably, the stretching rate is adjusted between 50 m/min and 500 m/min, and more preferably between 100 m/min and 400 m/min.

The diverging stretching device is schematically illustrated by Fig. 1 and 2 of US 5,043,036, and it is described in some detail in col.4, line 10 to col. 8, line 13 of this reference. Therefore Figs. 1 and 2 and the passage of US 5,043,036 specified are incorporated by reference into this specification.

The activatable zero strain composite laminate web of the present invention can be activated, if desired, by other activation methods including, for example, scoring or controlled localized stretching (also referred to as "ring rolling") as is disclosed, for example, in EP 0,521,883 or US 5,167,897.

Stretch-activating the composite laminate web of the present invention by non-destructive activation techniques provided, in particular, by the diverging disks stretching apparatus described above, provides, however, the unique advantage that the composite laminate web can be gently activated essentially without damaging or mechanically distorting the composite laminate. This is exemplified in Fig. 5d which shows a photograph of a portion of the composite laminate web of Example 6 which has been stretch-activated in the above diverging disks stretching device with subsequent relaxation. The edge area of the composite laminate portion which is referred to in Fig. 5d as "non-activated", had been clamped in the interacting guiding means such as the interacting ridges and grooves on the belts and pulleys and is therefore not activated. The remaining area of the composition laminate portion extending from the non-activated edge area in the cross-direction was stretched and is therefore labelled as "activated". The visual appearance of the non-activated edge area and the activated remainder area, respectively, of the stretch-activated composite laminate held in a relaxed state, is similar and/or essentially identical.

Subsequent to the stretch-activation the composite laminate web may be wound up in its relaxed state into a roll, or it can be processed in an in-line manufacturing process such as, for example, in an in-line diaper line. If desired, the non-activated edge area may be cut off from the activated composite laminate web prior to winding it in a roll or further processing it if a composite laminate web which is fully activated across its cross-directional extension, is desired. In other embodiments it may be desirable to maintain one or both non-activated edge areas of the composite laminate web. Sections of the activated composite laminate web which may be obtained by cutting the web in cross-direction may be used, for example, to provide elastic closure tabs suitable for hygienic disposable articles such as diapers. In such case, one of the non-activated edge areas may be used to apply the closure tab to the back side of the diaper (so-called "manufacturer's end") whereas a mechanical hook patch may be applied to the other non-activated edge area (so-called "user's end").

The activated composite laminates of the present invention exhibit favourable elastic properties and a highly appealing aesthetical appearance. Due to the relatively high elongation at break of the pre-bonded staple fiber nonwoven web, the composite laminate can be activated to essentially elastically recover in a broad elongation range without adversely affecting the mechanical integrity of the pre-bonded staple fiber nonwoven web.

The activated composite laminate web preferably exhibits when stretched in the first upload to an extension of 100 %, a retraction force at an elongation of 60 % during the first unload of at least 0.5 N/25.4 mm.

In an especially preferred embodiment the activatable zero strain composite laminate of the present invention comprises

- one or two pre-bonded nonwoven webs having, independently from each other, a bonding area of between 8 and 22 % with respect to the surface of the nonwoven web,
- an elastic laminate web having a core:skin thickness ratio of at least 6:1, whereby
- the one or two nonwoven webs are attached to the elastic laminate by adhesive layers with at least one of them being discontinuous.

In a more preferred embodiment both adhesive layers are discontinuous. The discontinuous adhesive layers preferably comprise essentially linear or curved strips extending in the machine direction.

It was found by the present inventors that the activated composite laminate obtained from such preferred activatable zero strain composite laminate is characterized by an especially attractive feeling to the touch combined with an attractive aesthetical appearance.

The activatable or activated composite laminate web materials of the present invention are suitable for use in disposable hygienic articles such as, for example, in diapers for baby or adult wearers, or in sanitary napkins. The activatable or activated composite laminate web materials may be used, for example, in closure tape tabs comprising an elastic bridge attached to two strips of a backing. One strip of the backing which is, for example, adhesively bonded to the diaper in order to secure the tape tab to the diaper, is often referred to as "manufacturer's end"; the other strip of the backing which may bear, for example, a mechanical fastening element, is gripped by the user when applying it to the wearer's body and is therefore often referred to as "user's end". The activatable or activated composite laminate web material is preferably sandwiched between and attached to the back strips.

Other diaper designs use so-called elastomeric ears which may be attached, for example, to diaper chassis of an essentially rectangular shape to provide side panels to the diaper. The activatable or activated composite laminate web materials of the present invention may be advantageously used in such side panels because they impart favourable wearing properties to the diaper.

The activatable or activated composite laminate web materials of the present invention may further be used in other portions of a diaper such as, for example, in the waistband area or in the scrotch area.

In hygienic applications, the composite laminate web material of the present invention may be used in the activatable or activated form, respectively. Using the activatable composite laminate web material of the present invention, for example, in closure tape tabs or side panels of diapers provides processing advantages but requires that the end user activates, for example, the closure tabs or side panels by stretching them which may require additional instructions for the user.

The activatable or activated composite laminate web material of the present invention is also useful for other applications such as, for example, in the textile or furniture industry.

The present invention will be further explained by the following Examples which are to be considered as illustrative and not limiting. Prior to this, some test methods are described which will be used in the Examples.

Test methods

*Thickness of the skin layers and the core layer of the elastic laminate web and core/skin thickness ratio*

The individual skin layers of the elastic laminate suitable in this invention are typically very thin (usually < 5 μm), and thus it can be difficult to measure their thicknesses by conventional photo-microscopy techniques. The thicknesses of the skin layers and the core layer of the elastic laminate web were therefore determined in a first measurement technique via weight and density calculations. A 2.54 cm $\times$ 15.24 cm strip of the elastic laminate was weighed to 4 decimal points on a Sartorius Analytic scale Model #A120S (Brinkman Instruments, Inc. Westbury, N. Y.) and then dissolved in toluene for 24 hours. The block copolymer elastomeric component and polystyrene component of the core layer are soluble in toluene, whereas, the polyolefin based skin layers are not soluble. The toluene solution was filtered through a Buchner™ funnel to collect the insoluble fraction on filter paper. The filter paper was dried for 1 hour at 70 °C, allowed to equilibrate to room temperature for 1 hour, and then weighed to 4 decimal points on the above mentioned Sartorius Analytic scale. By using the weight (before and after dissolving), the area, and the density, the sum of the thicknesses of the skin layers and the thickness of the core layer were calculated.

In an alternative way of measuring the individual thicknesses of the skin layers and the thickness of the elastic core layer were determined by fracturing the elastic laminate strips under liquid nitrogen, taking a photograph with an optical microscope, and measuring the respective layer thicknesses in the photograph with length measurement software.

The core/skin thickness ratio is obtained as the ratio of the thickness of the elastic core layer over the thickness of the skin layer (for elastic laminates with one skin layer) or over the sum of the thickness of both skin layers (for elastic laminates with two skin layers).

_Elongation at break and tensile strength at break_

Elongation at break and tensile strength at break were measured both for the nonwoven web and for the composite laminate web according to ISO 527-3 with the following modifications.

A 2.54 cm (MD) x 10.2 cm (CD) piece of the nonwoven web or the composite laminate web, respectively, cut in the cross direction, was mounted in a tensile testing machine (Zwick™ Model Z005 available from Zwick) with upper and lower jaws 2.54 cm apart. Line-contact jaws were used to minimize slip and breakage in the jaws. The jaws are then separated at a rate of 127 mm/min in the cross-direction. Elongation was measured and recorded in mm and percent and the force was measured and recorded in Newton when the corresponding sample broke.

The elongation in % as used herein is defined as:

$$\text{Elongation \%} = (\text{elongated length} - \text{original length})/ \text{original length x } 100$$

_Stretch activation of the acticvatable elastic laminate or the activatable composite laminate web, respectively, on the tensile testing device:_

A 2.54 cm (MD) x 10.2 cm (CD) piece of activatable elastic laminate or the activatable composite laminate web, respectively, cut in each case in the cross direction, was mounted in a tensile testing machine (Zwick™ Model Z005 available from Zwick) with upper and lower jaws 2.54 cm apart. Line-contact jaws were used to minimize slip and breakage in the jaws. The jaws are then separated at a rate of 127 mm/min in CD for a distance of 2.54 cm (100 %), 3.81 cm (150 %) and 5.08 cm (200 %), respectively.

_Hysteresis of the activatable elastic laminate or the activatable composite laminate web, respectively, and permanent set_

A 2.54 cm (MD) x 10.2 cm (CD) piece of the activatable elastic laminate or the activatable composite laminate web, respectively, cut in each case in the cross direction, was mounted in a tensile testing machine (Zwick™ Model Z005 available from Zwick) with upper and lower jaws 2.54 cm apart. Line-contact jaws were used to minimize slip and breakage in the jaws. The instrument cross head speed was set at a rate of 127mm/min. The elongation was set to the desired elongation of, for example, 100 % or 200 % in CD meaning a jaw movement of 25.4 mm or 50.8 mm, respectively. At the end point the jaw moved backwards without a holding time.

It is indicated in the Examples whether additional cycles were run after the first cycle whereby there was no holding time between the cycles. The upward movement of each cycle is termed as "upload" and the corresponding downward movement vas "unload" (the first cycle comprises, for example, the first upload curve and the first unload curve). The separation speed of the jaws (= stretching speed) during the testing was 127 mm/min.

The permanent set after the respective stretching cycle is defined as the point where the downwards or unload curve of such cycle goes through the base line (i.e. the x-axis). This is a measure for the increase in lengths or permanent deformation after stretch.

Examples

A: Elastic laminate webs

*Example 1*

A continuous coextrusion process was carried out to prepare a three-layer laminate with two outer inelastic skin layers of homo-polypropylene (PP) (melt flow index of 18 g/10 min) which is commercially available as 8069 Polypropylene from Total Petrochemicals, Feluy, Belgium, with a thickness of 3 $\mu$m each and an elastomeric core layer using styrene-isoprene-styrene (SIS) / polystyrene (PS) (70:30) polymers. The SIS used is a 100 % triblock styrene-isoprene-styrene which is supplied by Kraton Polymers, Pernis, The Netherlands, as Kraton D1114, Kraton 1160 SIS Rubber. The PS used has a melt flow rate of 13 cm$^3$/10 min and is available from Nova Chemicals, Carrington, UK, as Nova 3700 Crystal Grade PS. The core to skin ratio measured as described above is 8.2:1 as the elastic core layer was of 49 $\mu$m thickness and the non-elastic skin layers were 3 $\mu$m thick. One extruder was used to feed the elastomeric core layer material and a second extruder was used to feed the inelastic skin layer material into a 3-layer Cloeren™ feedblock, available from company Cloeren, Germany, and the resulting layered melt was extruded through a single manifold film die and cast onto a chill roll.

Samples were cut from the resulting activatable elastic laminate web in cross-direction so that they had an extension of 10.2 cm in the cross-direction and a width of 2.54 cm in the machine direction. A first sample was stretched in a first activation cycle in CD with an elongation speed of 127 mm/min on a Zwick tensile testing device as described above to an elongation of 40 %, and the sample was then relaxed. Fig. 3a shows a photograph of such sample in the relaxed state after the first activation cycle.

Further samples of the activatable elastic laminate were stretched likewise in their respective first activation cycle in CD with an elongation speed of 127 mm/min on a Zwick tensile testing device to an elongation of 80 %, 120 % and 160 %, respectively. Fig.'s 3c, 3e and 3g show photographs of such samples in each case in the relaxed state after the first activation cycle.

*Example 2*

Example 2 was prepared as described in Example I above with the difference that the thickness of the elastic core layer was 72 $\mu$m (instead of 49 $\mu$m in Example ) and that the two non-elastic PP skin layers had a thickness of 4 $\mu$m each (instead of 3 $\mu$m in Example 1).

Thus, the core/skin thickness ratio of the elastic laminate of Example 2 was 9.0:1.

*Example 3*

Example 3 was prepared as described in Example 1 above with the difference that the thickness of the elastic core layer was 91 $\mu$m (instead of 49 $\mu$m in Example ) and that the two non-elastic PP skin layers had a thickness of 4.4 $\mu$m each (instead of 3 $\mu$m in Example 1).

Thus, the core/skin thickness ratio of the elastic laminate of Example 2 was 10.3:1.

*Comparative Example 1*

Comparative Example 1 was prepared as described in Example 1 above with the difference that the thickness of the elastic core layer was 112 $\mu$m (instead of 49 $\mu$m in Example 1) and that the two non-elastic PP skin layers had a thickness of 12.5 $\mu$m each (instead of 3 $\mu$m in Example 1). Thus, the core/skin thickness ratio of the elastic laminate of Example 2 was 4.5:1.

Samples were cut from the resulting activatable elastic laminate web in the cross-direction so that they had an extension of 10.2 cm in the cross-direction and a width of 2.54 cm in the machine direction. A first sample was stretched in a first activation cycle in CD with an elongation speed of 127 mm/min on a Zwick tensile testing device as described above to an elongation of 40 %, and the sample was then relaxed. Fig. 3b shows a photograph of such sample in the relaxed state after the first activation cycle.

Further samples of the activatable elastic laminate were stretched likewise in their respective first activation cycle with an elongation speed of 127 mm/min on a Zwick tensile testing device to an elongation of 80 %, 120 % and 160 %, respectively. Fig.'s 3d, 3f and 3h show photographs of such samples in each case in the relaxed state after the first activation cycle.

A comparison of the photographs of Fig.'s 3b, 3d, 3f and 3h displaying top views of the partially or essentially fully activated elastic laminates of Comparative Example 1 with corresponding photographs of Fig.'s 3a, 3c, 3e and 3h displaying top views of the partially or essentially fully activated elastic laminates of Example 1, respectively, show that the activatable elastic laminate of Example 1 which is useful in the present invention, forms an essentially homogenous microtextured surface when stretched in the first upload in the cross-direction past the elastic limit of the skin layers. The microtexture can be macroscopically recognized in that the activatable elastic laminate when stretch-activated forms an essentially opaque appearance in comparison to the essentially clear appearance of the activatable laminate. The photograph of Fig. 3a which was taken in the relaxed state after an elongation to 40 % shows a relatively low degree of opacity while the photograph of Fig. 3c taken after an elongation of 80 % shows an intense opacity which does essentially not further intensify when stretching the elastic laminate of Example to 120 % or 160 %, respectively. It can be taken from this that the elastic laminate of Example 1 is only partially activated at an elongation of 40 % whereas an essentially full activation characterized by a fully developed microtextured surface of the skin layers, was reached at an elongation of about 80 %. The slight folds in Fig.'s 3a and 3h, respectively, result from an improper handling after stretching and did not evolve as a result of the stretching.

Contrary to this, it can be taken from the top view photographs of Fig.'s 3b, 3d, 3f and 3h that the activatable elastic laminate of Comparative Example 1 which is not useful in the present invention, displays macroscopic buckles and a distinctly inhomogeneous activation appearance which includes pronounced necking when stretched in the first upload in the cross-direction past the elastic limit of the skin layers. Fig. 3b shows that at a low elongation of 40 % the partially activated elastic laminate starts to neck at a location in the upper half of the sample. The photographs taken at an elongation of 80 %, 120 %  and 160 % show an increased necking behaviour and an activation of essentially only the upper half of the sample of the activatable elastic laminate. Fig. 3h shows that the sample of the activatable laminate was not yet fully activated at an elongation of 160 %. Fig. 4a shows a microphotograph magnified a hundred times which was taken in the essentially clear lower half of the sample of the activatable elastic laminate of Comparative Example 1 at an elongation of 80 % (see Fig. 3d). The photograph shows an essentially smooth surface structure corresponding to the surface structure of the non-stretched activatable elastic laminate of Comparative Example 1. Contrary to this, Fig. 4b shows a microphotograph magnified a hundred times which was taken in the opaque, activated upper half of the sample of the activatable elastic laminate of Comparative Example 1 at an elongation of 80 % (see Fig. 3d). The photograph shows a microtexture comprising individual folds and valleys.

Further samples were cut from the activatable elastic laminate webs of Examples 1 and 2 and of Comparative Example 1 in each case in cross-direction with a width of 2.54 cm in machine-direction. Each sample was stretched to an elongation of 200 % with a stretching speed of 127 mm/min and the first activation cycle was recorded as shown in Fig. 5a.

___: elastic laminate web of Example 1
___: elastic laminate web of Example 2
___: elastic laminate web of Comparative Example 1

It can be seen that the activatable elastic laminates of Examples 1 and 2 which are useful in the present invention, have stress-elongation plots which are similar in shape. Both stress-elongation plots show essentially no peak associated with the initial deformation and/or distortion of the skin layers and an almost constant force (Example 1) or a slightly increasing force (Example 2) is required for further stretching of the elastic laminates to an elongation of 200 %. The force required for stretch-activating the elastic laminate of Example 2 is higher than that required for the elastic laminate of Example I mainly because the elastic laminate of Example 2 comprises a core layer having a distinctly increased thickness over that of Example 1. Additionally, the skin layers of the elastic laminate of Example 2 are slightly thicker than the skin layers of the elastic laminate of Example 1.

Contrary to this, the stress-elongation plot of the activatable elastic laminate of Comparative Example I is markedly different in that this material exhibits a pronounced initial peak associated with the initial deformation and/or distortion of the skin layers. The force required for stretch-activating the elastic laminate of Comparative Example 1 is distinctly higher than that required for the elastic laminates of Example 1 or Example 2, respectively. The activated elastic laminate of Comparative Example 1 furthermore shows a permanent set of about 50 % which is distinctly higher than the permanent set of Example 1 and 2 (in each case about 20 %). This completely different behaviour of the elastic laminate of Comparative Example 1 in comparison to the elastic laminates of Examples 1 and 2, respectively, is mainly attributed to the distinctly increased thickness of the skin layers of the elastic laminate of Comparative Example 1, which translate into a distinctly lower core:skin thickness ratio of the elastic laminate of Comparative Example I in comparison to the elastic laminates of Examples 1 and 2, respectively. It can furthermore be seen that the activated elastic laminate of Example 1 and 2 show an elastic retraction force in the first unload at an elongation of 80 % of about 0.5 N/25.4 mm and about 1.5 N/25.4 mm, respectively, whereas the activated elastic laminate of Comparative Example 1 has an elastic retraction force of about 0 N/25.4 mm under these conditions.

Further samples were cut from the activatable elastic laminate webs of Example 3 and of Comparative Example 1 in each case in the cross-direction with a width of 2.54 cm in machine-direction. Each sample was stretched in CD to an elongation of 500 % with a stretching speed of 127 mm/min and the first and fifth activation cycles were recorded as shown in Fig. 5b.

> ___: elastic laminate web of Example 3
> ___: elastic laminate web of Comparative Example 1

B: Nonwoven webs

*Example 4*

The following nonwoven webs were provided:

- Carded nonwoven web Sawabond 4147 which is commercially available from Sandler AG, Schwarzenbach, Germany. Sawabond 4147 is made from staple fibers made from a homogeneous blend of PP and CoPP, having a pre-processing fibre elongation of about 250 - 350 %. The carded nonwoven web is thermo-bonded in a calander apparatus fitted with 11 % bonding area at a temperature of about 145 - 150 °C
  Further properties of Sawabond 4147:

  - basis weight about 22 g/m$^2$
  - fiber titer about 2.2 dtex (= 2.2g/100 m)
  - staple fiber length about 40 mm
  - about 25 essentially homogeneously distributed bonding points/cm$^2$
  - fiber orientation about 5:1 to 6:1 ((MD/CD)

- Carded nonwoven web Sawabond 4141 which is commercially available from Sandler AG, Schwarzenbach, Germany. Sawabond 4141 is made from staple fibers from a homogeneous blend of PP and CoPP, having a pre-processing elongation of about 250 - 350 %. The carded nonwoven web is thermo-bonded in a calander apparatus fitted with 21 % bonding area at a temperature of about 145 - 150 °C
  Further properties of Sawabond 4141:

  - basis weight about 24g/m$^2$
  - fiber titer about 2.2 dtex (= 2.2 g/100 m)
  - staple fiber length about 40 mm
  - about 21 bonding points/cm$^2$, distributed in a waffle-type pattern
  - fiber orientation about 5:1 to 6:1 (MD/CD)

- Carded nonwoven web Sawabond 4313 which is commercially available from Sandler AG, Schwarzenbach, Germany. Sawabond 4141 is made from S/C  bicofibres staple fibers, core component PP, sheath component PE, having a pre-processing elongation of about 200 - 350 %. The carded nonwoven web is thermo-bonded in a calander apparatus fitted with 26 % bonding rolls at a temperature of about 125-140 °C
  Further properties of Sawabond 4313:

  - basis weight about 22 g/m$^2$
  - fiber titer about 2.2 dtex (= 2.2 g/100 m)
  - staple fiber length about 40 mm
  - about 60 essentially homogeneously distributed bonding points/cm$^2$
  - fiber orientation about 5:1 to 6:1 (MD/CD)

- Spunbond nonwoven web Pegatex SSS which is commercially available from Pegas Nonwovens, Znojmo, Czech Republic.
  Further properties of Pegatex:

  - basis weight about 30 g/m$^2$
  - fiber titer about 2 dtex (= 2.2 g/100 m)

A 2.54 cm (MD) x 10.2 cm (CD) piece of each nonwoven web, cut in the cross-direction, was mounted in a tensile testing machine (Zwick™ Model Z005 available from Zwick) with upper and lower jaws 2.54 cm apart as described above. The jaws were then separated at a rate of 127 mm/min in CD, and the stress - elongation plots shown in Fig. 6 were recorded.

It can be seen from Fig. 6 that carded nonwoven webs Sawabond 4147 and Sawabond 4141 which show an elongation at break of more than 230 % and about 130 %, respectively, are useful in the present invention. Contrary to this, carded nonwoven web 4313 which shows an elongation at break of about 90 % is not useful in the present invention because it does not allow for a sufficient and/or desirable activation. The spunbond web Pegatex SSS requires distinctly higher stress values in comparison to all three carded webs. Pegatex SSS furthermore has an elongation at break of about 70 % and is thus not suitable for use in the present invention.

C: Composite laminate webs

*Example 5*

Two separate carded nonwoven webs Sawabond 4147 described above were provided. On one major side of each nonwoven web hotmelt adhesive HX20025-02 commercially available from Bostik Company Netherland B. V., Roosendaal, The Netherlands, was continuously coated full width at a basis weight of 5 g/m$^2$ onto the nonwoven web before its lamination to the elastic laminate web using a Porous Coat ® Applicator, commercially available from Company Nordson Engineering GmbH, Lüneburg, Germany.

The activatable elastic laminate web of Example 1 having a core/skin thickness ratio of 8.2:1 was provided.

The two-nonwoven webs and the activatable elastic laminate web were fed into a nip so that the elastic laminate web was sandwiched between the two nonwoven web layers with the hotmelt adhesive layers facing the skin layers of the activatable elastic laminate web. The nip was formed by a steel roll and another rubber roll without any additional temperature or cooling and the nonwoven webs and the activatable elastic laminate web were laminated under zero strain conditions.

The resulting activatable composite laminate was activated in the first activation cycle in a Zwick™ tensile testing machine as described above to an elongation of 100 % in CD using a stretching rate of 127 mm/min. The resulting activated composite laminate when held in a relaxed state after the first unload, exhibited a smooth and even surface similar to the surface of the nonwoven web. The nonwoven layer of the activated composite laminate web essentially did not show any macroscopic ruffles or foldings. In subsequent cycles the activated composite laminate could be stretched to 100 % and recovered essentially without any change in the visual appearance of the composite laminate web.

*Example 6*

Example 5 was repeated with the difference that the activatable elastic laminate web of Example 2 was used instead of the activatable elastic laminate web of Example 1.

The resulting activatable composite laminate was activated in the first activation cycle in a Zwick™ tensile testing machine as described above to an elongation of 100 % in CD using a stretching rate of 127 mm/min. The resulting activated composite laminate when held in a relaxed state after the first unload, exhibited a smooth and even surface similar to the surface of the nonwoven web. The nonwoven layer of the activated composite laminate web essentially did not show any macroscopic ruffles or foldings. In subsequent cycles the activated composite laminate could be stretched to 100 % and recovered essentially without any change in the visual appearance of the composite laminate web.

Fig. 5d provides a direct comparison of the appearance of the activated composite laminate web of Example 6 with respect to the appearance of the corresponding non-activated composite laminate web. The composite laminate web portion of Example 6 shown in Fig. 5d was activated in a diverging disk stretching apparatus as described above. The edge area of the composite laminate portion which is referred to in Fig. 5d as "non-activated", had been clamped in the interacting guiding means such as the interacting ridges and grooves on the belts and pulleys and is therefore not activated. The remaining area of the composite laminate portion extending from the non-activated edge area in the cross-direction was stretched and is therefore labeled as "activated". A visual inspection of the non-activated and activated areas, respectively, of the composite laminate portion of Fig. 5d shows that the appearance of these two areas is essentially identical.

*Example 7*

Example 5 was repeated with the difference that the activatable elastic laminate web of Example 3 was used instead of the activatable elastic laminate of Example 1.

The resulting activatable composite laminate was activated in the first activation cycle in a Zwick™ tensile testing machine as described above to an elongation of 100 % in CD using a stretching rate of 127 mm/min. The resulting activated composite laminate when held in a relaxed state after the first unload, exhibited a smooth and even surface similar to the surface of the nonwoven web. The nonwoven layer of the activated composite laminate web essentially did not show any macroscopic ruffles or foldings. In subsequent cycles the activated composite laminate could be stretched to 100 % and recovered essentially without any change in the visual appearance of the composite laminate web.

*Fig. 5c* shows the first three activation cycles in CD for the composite laminate webs of Examples 5-7 recorded at a stretch rate of 127 mm/min as described above.

___ : composite laminate web of Example 5 (comprising the elastic laminate web of Example 1)
___ : composite laminate web of Example 6 (comprising the elastic laminate web of Example 2)
___ : composite laminate web of Example 7 (comprising the elastic laminate web of Example 3)

It can be seen that the activatable composite laminates of Examples 5 - 7 have stress-elongation plots which are similar in shape. The force required for stretch-activating the composite laminate of Example 6 and 7 is higher than that required for the composite laminate of Example 5 mainly because the elastic laminates of Examples 6 and 7 comprise core layers having an increased thickness over that of Example 5. Additionally, the elastic laminates of Examples 6 and 7, respectively, comprise skin layers having an increased thickness over the skin layers of Example 5.

*Example 8*

Example 5 was repeated with the difference that hot melt adhesive HX20025-02 was coated onto the two carded nonwoven webs Sawabond 4147 in a discontinuous fashion applying straight, parallel adhesive strips extending in the machine direction and having a width of 2 mm in the cross-direction. The adhesive-free areas between the adhesive strips had a width in the cross-direction of 1 mm so that 66 % of the respective surfaces of the nonwoven webs were adhesive coated. The adhesive was coated at a basis weight of 5 g/m$^2$ as in Example 5. Strip-coating of the adhesive was effected by a modified space plate.

The resulting activatable composite laminate was activated in the first activation cycle in CD in a Zwick™ tensile testing machine as described above to an elongation of 100 % using a stretching rate of 127 mm/min. The resulting activated composite laminate when held in a relaxed state after the first unload, exhibited a smooth and even surface which appeared to be fluffier and fuller to the touch as compared to the appearance of the activated composite laminate of Example 5. This is attributed to the formation of small ruffles in the non-adhesive coated areas between the adhesive strips. The non-woven layers were intact and not broken.

In subsequent cycles the activated composite laminate could be stretched to an elongation of 100 % and recovered essentially without any change in the visual appearance of the composite laminate web.

*Example 9*

Example 8 was repeated with the difference that the width of the adhesive strips in the cross-direction was 1 mm and the width of the adhesive-free areas between such strips in the cross-direction was 2 mm.

The resulting activatable composite laminate was activated in the first activation cycle in CD in a Zwick™ tensile testing machine as described above to an elongation of 100 % using a stretching rate of 127 mm/min. The resulting activated composite laminate when held in a relaxed state after the first unload, exhibited an essentially smooth and even surface comprising lines extending in the machine direction. The nonwoven was, however, intact and not broken at such lines. The activated composite laminate appeared to be fluffier and fuller to the touch as compared to the appearance of the activated composite laminate of Example 5.

In subsequent cycles the activated composite laminate could be stretched to an elongation of 100 % and recovered essentially without any change in the visual appearance of the composite laminate web.

The following table 1 compares the stretch-elongation behavior of the continuously adhesive bonded laminate of Example 5 with the discontinuously adhesive bonded laminates of Examples 8 and 9.

*Table 1: Properties of the continuously adhesive bonded laminate of Example 5 with the discontinuously adhesive bonded laminates of Examples 8 and 9*

| Example | 5 | 8 | 9 |
|---|---|---|---|
| Stress [N/inch] at an elongation of 5%inCD | 2.5 | 0.5 | 1.0 |
| Elongation [%] in CD at a stress of 5 N/25.4 mm | 6 | 33 | 32 |
| Elongation [%] at a stress of 10 N/25.4 mm in CD | 9 | 53 | 52 |

It can be seen from table 1 that the forces required to achieve an elongation of 5 % in CD are significantly lower for the strip-bonded composite laminates of Examples 8 and 9 in comparison to the full width adhesive coated composite laminate of Example 5.

List of reference numbers

[0138]

| 1 | elastic core layer |
| 2 | skin layer |
| 3 | adhesive layer |
| 3a | adhesive strip |
| 4 | nonwoven layer |
| 10 | activatable composite laminate |
| 20 | activated composite laminate |

**Claims**

1. An activatable zero strain composite laminate web comprising an activatable elastic laminate web having an elastic core layer and at least one skin layer which is less elastic than the core layer, and at least one pre-bonded staple fiber nonwoven web which is attached to one of the skin layers of the elastic laminate web, the at least one staple fiber nonwoven web having an elongation at break of at least 100 % in the cross-direction and said activatable elastic laminate web forming an essentially homogeneous microtextured surface when stretched in the first upload in the cross-direction past the elastic limit of the one or more skin layers.

2. Activatable composite laminate web according to claim 1 wherein the staple fiber nonwoven web is a carded non-woven web.

3. Activatable composite laminate web according to any of the preceding claims wherein the pre-bonded staple non-woven web exhibits a bonding area of between 8 and 22 % with respect to the surface of the web.

4. Activatable composite laminate web according to any of the preceding claims wherein the ratio of the thickness of the core layer of the elastic laminate web over the thickness of the at least one skin layer or the sum of the skin layers, respectively, of such web is at least 6:1.

5. Activatable composite laminate web according to any of the preceding claims wherein the elastic laminate web when stretched in the first upload to an extension of 200 %, has a permanent set during the first unload of less than 30 %.

6. Activatable composite laminate web according to any of the preceding claims wherein the elastic core comprises one or more elastomers selected from the group consisting of block copolymers including styrene/isoprene/styrene (SIS), styrene/butadiene/styrene (SBS) or styrene/ethylene/butylene/styrene (SEBS) block copolymers elastomeric polyurethanes, elastomeric ethylene copolymers including elastomeric ethylene vinyl acetates, ethylene/propylene copolymer elastomers and ethylene/propylene diene copolymer elastomers, and blends of these.

7. Activatable composite laminate web according to any of the preceding claims wherein the at least one skin layer comprises one or more polymers selected from the group consisting of polyolefin and polyolefin copolymers including polyethylene, polypropylene, polybutylene and polyethylene-polypropylene, polyamide including nylon, polyester including polyethylene terephthalate, and blends thereof.

8. Activatable composite laminate web according to any of the preceding claims wherein the at least one staple fiber nonwoven layer is attached to the elastic laminate by thermo bonding, ultrasonic bonding or adhesive bonding.

9. Activatable composite laminate web according to claim 8 wherein the adhesive bonding is effected by discontinuous adhesive layers.

10. Activatable composite laminate web according to claim 9 containing adhesive strips wherein the adhesive strips exhibit a width in cross-direction of between 0.5 and 3 mm and wherein the adhesive-free space between the adhesive strips is between 0.5 and 3 mm.

11. An activatable zero strain composite laminate web comprising an activatable elastic laminate web having an elastic core layer and at least one skin layer which is less elastic than the core layer, and at least one pre-bonded staple fiber nonwoven web which is attached to one of the skin layers of the elastic laminate web, the at least one staple fiber nonwoven web having an elongation at break of at least 100 % in the cross-direction and a bonding area of between 8 and 22 % with respect to the surface of the nonwoven web, said activatable elastic laminate web having a ratio of the thickness of the core layer of the elastic laminate web over the thickness of the at least one skin layer or the sum of the skin layers, respectively, of such web of at least 6:1, wherein at least one staple fiber nonwoven layer is attached to the elastic laminate by a discontinuous adhesive layer.

12. Activated composite laminate web obtainable by stretching of an activatable composite laminate web according to any of claims 1 to 11 past the elastic limit of the one or more skin layers but below the elongation at break of the staple fiber nonwoven web.

13. Activated composite laminate portion obtainable by cutting a portion from the activated composite laminate web of claim12 in cross-direction.

14. Closure tape tab or side panel each comprising a fastening means and an activated composite laminate portion according to claim 13.

15. Method of preparing an activatable composite laminate according to any of claims 1 to 11 comprising the steps of

(i) providing an activatable elastic laminate web having an elastic core layer and at least one skin layer which is less elastic than the core layer, said activatable elastic laminate web forming an essentially homogeneous microtextured surface when being stretched in the first upload in cross-direction past the elastic limit of the one or more skin layers;
(ii) providing at least one staple nonwoven web having an elongation at break of at least 100 % in cross-direction; and
(iii) attaching said stable nonwoven web to said elastic laminate web whereby the activatable elastic laminate web is held in an essentially untensioned state.

16. Method of preparing an activated composite laminate web according to claims 12 and 13 comprising the steps of

(i) providing an activatable composite laminate web according to claims I - 10; and
(ii) stretching such activatable composite laminate web in the cross direction past the elastic limit of the one or more skin layers but below the elongation at break of the staple fiber nonwoven layer web.

**Patentansprüche**

1. Aktivierbare spannungslose Verbundlaminatbahn, aufweisend eine aktivierbare elastische Laminatbahn, welche eine elastische Kernschicht und mindestens eine Außenschicht, die weniger elastisch als die Kernschicht ist, und mindestens eine vorgebundene Stapelfaser-Vliesbahn aufweist, welche an einer der Außenschichten der elastischen Laminatbahn befestigt ist, wobei die mindestens eine Stapelfaser-Vliesbahn eine Bruchdehnung von mindestens 100 % in Querrichtung aufweist und die aktivierbare elastische Laminatbahn eine im Wesentlichen homogene mikrotexturierte Fläche bildet, wenn sie bei der ersten Belastung in der Querrichtung jenseits der Elastizitätsgrenze der einen oder der mehreren Außenschichten gedehnt wird.

2. Aktivierbare Verbundlaminatbahn nach Anspruch 1, wobei die Stapelfaser-Vliesbahn eine kardierte Vliesbahn ist.

3. Aktivierbare Verbundlaminatbahn nach einem der vorhergehenden Ansprüche, wobei die vorgebundene Stapelfaser-Vliesbahn einen Verbindungsbereich von 8 % bis 22 %, bezogen auf die Fläche der Bahn, aufweist.

4. Aktivierbare Verbundlaminatbahn nach einem der vorhergehenden Ansprüche, wobei das Verhältnis der Dicke der Kernschicht der elastischen Laminatbahn zu der Dicke der mindestens einen Außenschicht bzw. der Summe der Außenschichten einer solchen Bahn mindestens 6:1 beträgt.

5. Aktivierbare Verbundlaminatbahn nach einem der vorhergehenden Ansprüche, wobei die elastische Laminatbahn, wenn sie bei der ersten Belastung auf eine Dehnung von 200 % gedehnt wird, während der ersten Entlastung einen Verformungsrest von weniger als 30 % aufweist.

6. Aktivierbare Verbundlaminatbahn nach einem der vorhergehenden Ansprüche, wobei der elastische Kern ein oder mehrere Elastomere aufweist, die aus der Gruppe ausgewählt sind, die aus Blockcopolymeren, z.B. Styrol/Isopren/Styrol(SIS)-, Styrol/Butadien/Styrol(SBS)- oder Styrol/Ethylen/Butylen/Styrol(SEBS)-Blockcopolymeren, elastomeren Polyurethanen, elastomeren Ethylen-Copolymeren, z.B. elastomeren Ethylen-Vinylacetaten, Ethylen/Propylen-Copolymer-Elastomeren und Ethylen/Propylen-Dien-Copolymer-Elastomeren und Mischungen dieser besteht.

7. Aktivierbare Verbundlaminatbahn nach einem der vorhergehenden Ansprüche, wobei die mindestens eine Außenschicht ein oder mehrere Polymere aufweist, die aus der Gruppe ausgewählt sind, die aus Polyolefinen und Polyolefin-Copolymeren, z.B. Polyethylen, Polypropylen, Polybutylen und Polyethylen-Polypropylen, Polyamid, z.B. Nylon, Polyester, z.B. Polyethylenterephthalat, und Mischungen dieser besteht.

8. Aktivierbare Verbundlaminatbahn nach einem der vorhergehenden Ansprüche, wobei die mindestens eine Stapelfaser-Vliesschicht durch Thermobonding, Ultraschall-Bonding oder Klebstoffverbindung an dem elastischen Laminat befestigt ist.

9. Aktivierbare Verbundlaminatbahn nach Anspruch 8, wobei die Klebstoffverbindung durch diskontinuierliche Klebstoffschichten bewirkt wird.

10. Aktivierbare Verbundlaminatbahn nach Anspruch 9, welche Klebstoffstreifen enthält, wobei die Klebstoffstreifen eine Breite in Querrichtung von 0,5 mm bis 3 mm aufweisen und wobei der klebstofffreie Raum zwischen den Klebstoffstreifen 0,5 mm bis 3 mm beträgt.

11. Aktivierbare spannungslose Verbundlaminatbahn, aufweisend eine aktivierbare elastische Laminatbahn, welche eine elastische Kernschicht und mindestens eine Außenschicht, die weniger elastisch als die Kernschicht ist, und mindestens eine vorgebundene Stapelfaser-Vliesbahn aufweist, welche an einer der Außenschichten der elastischen Laminatbahn befestigt ist, wobei die mindestens eine Stapelfaser-Vliesbahn eine Bruchdehnung von mindestens 100 % in Querrichtung und einen Verbindungsbereich von 8 % bis 22 %, bezogen auf die Fläche der Vliesbahn, aufweist, wobei die aktivierbare elastische Laminatbahn ein Verhältnis der Dicke der Kernschicht der elastischen Laminatbahn zu der Dicke der mindestens einen Außenschicht bzw. der Summe der Außenschichten einer solchen Bahn von mindestens 6:1 aufweist, wobei die mindestens eine Stapelfaser-Vliesschicht durch eine diskontinuierliche Klebstoffschicht an dem elastischen Laminat befestigt ist.

12. Aktivierte Verbundlaminatbahn, zu erhalten durch Dehnen einer aktivierbaren Verbundlaminatbahn nach einem der Ansprüche 1 bis 11 jenseits der Elastizitätsgrenze der einen oder der mehreren Außenschichten, jedoch unterhalb der Bruchdehnung der Stapelfaser-Vliesbahn.

13. Aktivierter Verbundlaminatabschnitt, zu erhalten durch Herausschneiden eines Abschnitts aus der aktivierten Verbundlaminatbahn nach Anspruch 12 in Querrichtung.

14. Verschlussbandlasche oder Seitenteil, jeweils aufweisend ein Befestigungsmittel und einen aktivierten Verbundlaminatabschnitt nach Anspruch 13.

15. Verfahren zur Herstellung eines aktivierbaren Verbundlaminats nach einem der Ansprüche 1 bis 11, aufweisend die Schritte

   (i) Bereitstellen einer aktivierbaren elastischen Laminatbahn, aufweisend eine elastische Kernschicht und mindestens eine Außenschicht, die weniger elastisch als die Kernschicht ist, wobei die aktivierbare elastische Laminatbahn eine im Wesentlichen homogene mikrotexturierte Fläche bildet, wenn sie bei der ersten Belastung in Querrichtung jenseits der Elastizitätsgrenze der einen oder der mehreren Außenschichten gedehnt wird;
   (ii) Bereitstellen mindestens einer Stapelfaser-Vliesbahn, welche eine Bruchdehnung von mindestens 100 % in Querrichtung aufweist; und
   (iii) Befestigen der Stapelfaser-Vliesbahn an der elastischen Laminatbahn, wodurch die aktivierbare elastische Laminatbahn in einem im Wesentlichen spannungsfreien Zustand gehalten wird.

**16.** Verfahren zur Herstellung einer aktivierten Verbundlaminatbahn nach Anspruch 12 und 13, aufweisend die Schritte

(i) Bereitstellen einer aktivierbaren Verbundlaminatbahn nach Anspruch 1 bis 10; und
(ii) Dehnen einer solchen aktivierbaren Verbundlaminatbahn in der Querrichtung jenseits der Elastizitätsgrenze der einen oder der mehreren Außenschichten, jedoch unterhalb der Bruchdehnung der Stapelfaser-Vliesschichtbahn.

**Revendications**

**1.** Toile stratifiée composite activable à tension zéro, comprenant une toile stratifiée élastique activable possédant une couche de coeur élastique et au moins une couche de peau, qui est moins élastique que la couche de coeur, et au moins une toile non tissée de fibres discontinues pré-collées qui est attachée à l'une des couches de peau de la toile stratifiée élastique, la au moins une toile non tissée de fibres discontinues ayant un allongement à la rupture d'au moins 100% dans le sens travers et ladite toile stratifiée élastique activable formant une surface micro-texturée essentiellement homogène lorsqu'elle est étirée dans une première charge en travers après la limite élastique de l'une ou de plusieurs couches de peau.

**2.** Toile stratifiée composite activable selon la revendication 1 dans laquelle la toile non tissée à fibres discontinues est une toile non tissée cardée.

**3.** Toile stratifiée composite activable selon l'une quelconque des revendications précédentes dans laquelle la toile non tissée à fibre discontinues pré-collée présente une aire de collage entre 8 et 22 % par rapport à la surface de la toile.

**4.** Toile stratifiée composite activable selon l'une quelconque des revendications précédentes dans laquelle le ratio entre l'épaisseur de la couche de coeur de la toile stratifiée élastique et l'épaisseur de l'au moins une couche de peau, ou de l'ensemble des couches de peau, respectivement, d'une telle toile est au moins 6 : 1.

**5.** Toile stratifiée composite activable selon l'une quelconque des revendications précédentes dans laquelle la toile stratifiée élastique, lorsqu'étirée dans la première charge jusqu'à une extension de 200 %, possède une déformation permanente pendant la première décharge inférieur à 30 %.

**6.** Toile stratifiée composite activable selon l'une quelconque des revendications précédentes dans laquelle le coeur élastique comprend un ou plusieurs élastomères choisis dans le groupe constitué de copolymères blocs incluant des élastomères copolymères blocs polyuréthanes styrène/isoprène/styrène (SIS), styrène/butadiène/styrène (SBS) ou styrène/éthylène/butylène/styrène (SEBS), des élastomères copolymères d'éthylène incluant des élastomères éthylène acétate de vinyle, des élastomères de copolymères éthylène/propylène et des élastomères de copolymères diène éthylène/propylène, et des mélanges de ceux-ci.

**7.** Toile stratifiée composite activable selon l'une quelconque des revendications précédentes dans laquelle l'au moins une couche de peau comprend un ou plusieurs polymères choisis dans le groupe formé par les polyoléfines et les copolymères de polyoléfines incluant le polyéthylène, le polypropylène, le polybutylène et le polyéthylène-polypropylène, le polyamide incluant le nylon, le polyester incluant le polyéthylène téréphtalate, et les mélanges de ceux-ci.

**8.** Toile stratifiée composite activable selon l'une quelconque des revendications précédentes dans laquelle au moins l'une des couches non tissées de fibres discontinues est attachée au stratifié élastique par thermocollage, collage ultrasonique ou collage adhésif.

**9.** Toile stratifiée composite activable selon la revendication 8 dans laquelle le collage adhésif est réalisé par des couches adhésives discontinues.

**10.** Toile stratifiée composite activable selon la revendication 9 contenant des bandes adhésives, les bandes adhésives ayant une largeur dans la direction transverse entre 0,5 et 3 mm et l'espace libre de tout adhésif entre les bandes adhésives étant entre 0,5 et 3 mm.

**11.** Toile stratifiée composite activable à tension zéro comprenant une toile stratifiée élastique activable possédant une couche de coeur élastique et au moins une couche de peau, qui est moins élastique que la couche de coeur, et au moins une toile non tissée de fibres discontinues pré-collées qui est attachée à celle des couches de peau de la toile stratifiée élastique, la au moins une toile non tissée de fibres discontinues ayant un allongement à la rupture d'au moins 100 % dans le sens travers et une aire de collage entre 8 et 22 % par rapport à la surface de la toile non tissée, ladite toile stratifiée élastique activable ayant un ratio entre l'épaisseur de la couche de coeur de la toile stratifiée élastique et l'épaisseur d'au moins une couche de peau, ou de l'ensemble des couches de peau, respectivement, d'une telle toile d'au moins 6 : 1, au moins une couche non tissée de fibres discontinues étant fixée au stratifié élastique par une couche adhésive discontinue.

12. Toile stratifiée composite activée pouvant être obtenue par étirement d'une toile stratifiée composite activable, selon l'une quelconque des revendications 1 à 11, après la limite élastique de l'une ou plusieurs couches de peau, mais en dessous de l'allongement à la rupture de la toile non tissée en fibres discontinues.

13. Partie stratifiée composite activée pouvant être obtenue en coupant par le travers une partie de la toile stratifiée composite activée selon la revendication 12.

14. Onglet de fermeture à bande, ou panneau latéral, comprenant chacun un moyen de fixation et une partie stratifiée composite activée selon la revendication 13.

15. Procédé de préparation d'un stratifié composite activable selon l'une quelconque des revendications 1 à 11 comprenant les étapes de

(i) fourniture d'une toile stratifiée élastique activable possédant une couche de coeur élastique et au moins une couche de peau, qui est moins élastique que la couche de coeur, ladite toile stratifiée élastique activable formant une surface micro-texturée essentiellement homogène quand elle est étirée dans la première charge en travers après la limite élastique de l'une ou plusieurs couches de peau ;
(ii) fourniture d'au moins une toile non tissée à fibre discontinues ayant un allongement à la rupture d'au moins 100 % en sens travers ; et
(iii) fixation de ladite toile non tissée à fibres discontinues à ladite toile stratifiée élastique, moyennant quoi, la toile stratifiée élastique activable est gardée dans un état essentiellement de non tension.

16. Procédé de préparation d'une toile stratifiée composite activée selon les revendications 12 et 13 comprenant les étapes de

(i) fourniture d'une toile stratifiée composite activable selon les revendications 1 à 10 ; et
(ii) étirement en travers d'une telle toile stratifiée composite activable après la limite élastique de l'une ou de plusieurs couches de peau, mais en dessous de l'allongement à la rupture de la toile de la couche non tissée en fibres discontinues.

*Fig. 1*

*Fig. 1a*

*Fig. 1b*

*Fig. 2*

*Fig. 3a*

*Fig. 3b*

*Fig. 3c*

*Fig. 3d*

*Fig. 3e*

*Fig. 3f*

*Fig. 3g*

*Fig. 3h*

*Fig. 4a*

*Fig. 4b*

Fig. 5a

Fig. 5b

Fig. 5c

Fig. 5d

*Fig. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5501679 A
- EP 0521883 A
- US 6159584 A
- US 5167897 A
- US 7078089 B
- WO 9616216 A
- US 3265765 A
- US 3562 A
- US 356 A
- US 3700633 A
- US 4116917 A
- US 4156673 A
- US 6476289 B
- US 5422172 A
- US 5043036 A